(19) Europäisches Patentamt
European Patent Office
Office européen des brevets

(11) Numéro de publication : **0 453 690 B1**

(12) # FASCICULE DE BREVET EUROPEEN

(45) Date de publication du fascicule du brevet : **22.06.94 Bulletin 94/25**

(51) Int. Cl.$^5$ : **C07C 51/377,** C07C 53/16, B01J 27/045

(21) Numéro de dépôt : **90401094.9**

(22) Date de dépôt : **24.04.90**

(54) **Procédé et catalyseur de déshalogénation d'acides carboxyliques alphahalogènes.**

(43) Date de publication de la demande : **30.10.91 Bulletin 91/44**

(45) Mention de la délivrance du brevet : **22.06.94 Bulletin 94/25**

(84) Etats contractants désignés : **AT BE CH DE DK ES FR GB GR IT LI LU NL SE**

(56) Documents cités :
**DE-B- 1 915 037**
**FR-A- 2 582 645**
**GB-A- 1 419 603**
**US-A- 2 863 917**
**US-A- 4 288 422**

(73) Titulaire : **ELF ATOCHEM S.A.**
**4 & 8, Cours Michelet**
**La Défense 10**
**F-92800 Puteaux (FR)**

(72) Inventeur : **Correia, Yves**
**Les Lauzières**
**F-04160 Chateau-Arnoux (FR)**
Inventeur : **Bertucci, Michel**
**166 Boulevard de la Croix Rousse**
**F-69001 Lyon (FR)**

EP 0 453 690 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé et un catalyseur de deshalogénation d'acides carboxyliques alphahalogénés, elle est particulièrement utile pour éliminer l'acide dichloracétique (ADCA) contenu dans l'acide monochloracétique (AMCA). La synthèse de l'acide monochloracétique à l'échelle industrielle se fait par la chloration de l'acide acétique, mais il se forme inévitablement de l'acide dichloracétique et parfois un peu d'acide trichloracétique. On obtient donc à l'issue de la chloration un mélange constitué d'acide monochloracétique, d'acide dichloracétique, de traces de trichloracétique et d'acide acétique qui n'a pas réagi. Compte-tenu de la proximité des points d'ébullition de l'AMCA (189°C) et de l'ADCA (194°C), il est pratiquement impossible de les séparer par distillation. Par contre, il est très simple d'hydrogéner ce mélange pour convertir l'ADCA en AMCA selon la réaction :

$$CHCl_2COOH + H_2 \text{--->} CH_2ClCOOH + HCl$$

Cette hydrogénation n'est pas totalement sélective et on observe aussi une rétrogradation d'AMCA en acide acétique :

$$CH_2ClCOOH + H_2 \text{--->} CH_3COOH + HCl$$

Cette réaction se fait avec un catalyseur et sous-produit de l'acétaldéhyde qui a l'inconvénient de générer des produits de condensation.

Le brevet FR 1 581 391 décrit un tel procédé basé sur un catalyseur constitué de silice sous forme de cylindres de longueur 8 mm et 3,5 mm de diamètre, ayant une teneur en palladium de 0,5 % en poids.

Le brevet FR 2 039 987 décrit un procédé du même genre que le précédent mais en ajoutant soit des sels ou des oxydes de métaux alcalins ou alcalino-terreux ou des terres rares, soit un composé organique connu sous le nom d'accepteur de protons pouvant être par exemple la triéthylamine, la pyridine, la pipéridine, la glycine, l'urée, la triphénylphosphine, le butyraldéhyde, la diisobutylcétone, l'acétate de butyle ou l'acide cyanhydrique. Cet activateur du catalyseur s'introduit dans le mélange à hydrogéner et peut être récupéré par distillation du mélange hydrogéné. On peut le recycler dans le mélange à hydrogéner. La demanderesse a trouvé un procédé dans lequel on modifie l'action du catalyseur par l'utilisation d'un activateur, cette activation du catalyseur est durable et s'observe sous une forme légèrement modifiée quand on cesse d'injecter l'activateur dans le mélange à hydrogéner.

Un deuxième but de l'invention est d'hydrogéner le plus sélectivement possible les acides di- et tri-alphahalogénés.

Un troisième but de l'invention est de réduire la sous-production des aldéhydes correspondants aux acides carboxyliques alphahalogénés.

La présente invention est donc un procédé de deshalogénation d'acides carboxyliques alphahalogénés, ou de leurs esters, par l'hydrogène en présence d'un catalyseur à base d'un métal précieux du groupe VIII caractérisé en ce qu'on effectue ladite deshalogénation en présence de soufre ou de composés du soufre.

Bien que l'invention puisse s'appliquer à tous les acides carboxyliques alphahalogénés, elle est surtout utilisée pour les acides de formule :

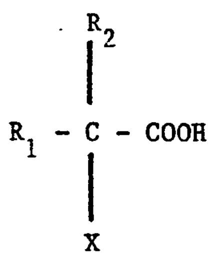

(I)

dans laquelle X est le chlore ou le brome, $R_1$ et $R_2$ sont identiques ou différents et représentent X, H, un radical alkyle linéaire ou ramifié ayant de 1 à 12 carbones ou un radical cycloalkyle ayant de 3 à 12 carbones. L'invention s'applique aussi aux esters des acides de formule (I). Ce sont de préférence les esters aliphatiques ayant de 1 à 10 carbones, et de préférence 1 à 5 atomes de carbone.

Selon l'invention on peut effectuer la deshalogénation d'un acide ou d'un mélange d'acides. Ces acides peuvent être aussi en mélange avec un solvant. Les métaux précieux du groupe VIII de la classification périodique des éléments sont le ruthénium, le rhodium, le palladium, l'osmium, l'iridium et le platine. On utilise avantageusement les trois premiers et de préférence le palladium. Ces métaux peuvent être utilisés seuls, en alliages ou en mélange entre eux. Ils peuvent être utilisés tels quels ou déposés sur un support par exemple du charbon, de la silice, du carbure de silicium ou du carbure de bore. On peut utiliser le catalyseur sous forme

de fines particules, c'est-à-dire qu'on mélange les acides et le catalyseur, on ajoute le soufre et on introduit l'hydrogène. Selon les applications des acides deshalogénés, il est parfois nécessaire de séparer en fin de deshalogénation le catalyseur des acides. Selon une forme avantageuse de l'invention, le catalyseur est disposé en lit fixe ou en lit fluide dans une capacité et les acides à deshalogéner, l'hydrogène et le soufre sont introduits dans cette capacité. Il n'est donc pas nécessaire en fin de deshalogénation de séparer le catalyseur. De préférence on utilise un cata en lit fixe et on opère en continu.

Le métal précieux est avantageusement déposé sur un charbon de grande surface à raison de 0,3 à 1 % en poids du catalyseur, c'est-à-dire du charbon plus du métal et il est réparti en surface du charbon. On entend par charbon de grande surface un charbon d'environ 600 $m^2/g$ et pouvant aller jusqu'à 1300 $m^2/g$.Ce charbon se présente sous forme de petits cylindres extrudés ou d'une poudre d'environ 50 $\mu$. La quantité de catalyseur est choisie par l'homme de métier.

Avantageusement on ajoute le soufre ou les composés du soufre dans les acides à deshalogéner. Les composés du soufre peuvent être $SCl_2$, $S_2Cl_2$, $CS_2$, du thiophène, des produits oxygénés du soufre. Le soufre ou les composés du soufre sont ajoutés à l'état de traces jusqu'à 1 g par Kg d'acide ou du mélange contenant les acides à hydrogéner, et de préférence entre 0,1 et 200 mg/Kg exprimé en soufre. Il est avantageux d'opérer le procédé de l'invention avec les acides en phase liquide. Bien qu'on puisse opérer à toute température, il est avantageux d'être entre la température à laquelle les acides sont liquides et 200°C, et de préférence entre 100 et 180°C. Si nécessaire, on peut mettre le ou les acides dans un solvant pour pouvoir se placer dans cette zone de température préférée.

On peut opérer à la pression atmosphérique ou jusqu'à 5 bars. L'effet de la pression est d'augmenter la cinétique de réaction, comme les acides sont corrosifs et aussi le milieu réactionnel de l'invention, il n'est pas judicieux de dépasser une pression de l'ordre de 5 bars.

L'invention est particulièrement utile pour purifier des acides carboxyliques monoalphahalogénés $R_1CHXCOOH$ impurs, $R_1$ avant la signification précédente. Ces acides sont préparés par halogénation de l'acide correspondant $R_1CH_2COOH$, on obtient un mélange de $R_1CHXCOOH$, de $R_1CX_2COOH$, d'acide $R_1CH_2COOH$ qui n'a pas été transformé et parfois des traces de $CX_3COOH$ dans le cas particulier de l'acide $CH_3COOH$.

On pourrait d'abord séparer $R_1CH_2COOH$ de ce mélange mais il est plus simple d'hydrogéner d'abord

$$R_1CX_2COOH + H_2 \text{ ---> } R_1CHXCOOH + HX$$

et séparer ensuite, puisque inévitablement une partie de $R_1CHXCOOH$ est rétrogradée en acide selon :

$$R_1CHXCOOH + H_2 \text{ ---> } R_1CH_2COOH + HX$$

Il suffit ensuite de distiller le mélange de $R_1CHXCOOH$, de $R_1CH_2COOH$ et d'HX pour obtenir $R_1CHXCOOH$ relativement pur.

L'invention s'applique en particulier à la purification de l'acide monochloracétique.

On démarre la deshalogénation avec le seul catalyseur sans le soufre, c'est-à-dire l'art antérieur, et la demanderesse a constaté que quelques heures après l'introduction du soufre, et alors qu'on continue à l'introduire, on observait une conversion de $R_1CX_2COOH$ légèrement plus faible à la même température, une beaucoup plus faible rétrogradation et une forte diminution de l'aldéhyde sous-produit $R_1CH_2CHO$.

La rétrogradation est le rapport entre le nombre d'ions $X^-$ dans l'acide purifié, c'est-à-dire ceux provenant de HX au nombre théorique de X à enlever de $R_1CX_2COOH$ (et éventuellement $CX_3COOH$) pour le convertir en $R_1CHXCOOH$. Sauf exception de $CX_3COOH$, la rétrogradation minimum est de 1. Cette rétrogradation est le plus souvent entre 1,4 et 3,4.

La demanderesse a constaté avec surprise qu'en arrêtant l'introduction du soufre on continuait d'en voir les effets sous une forme peu modifiée, c'est-à-dire que la conversion de $R_1CX_2COOH$ remonte et dépasse celle de l'art antérieur, la rétrogradation remonte et se situe entre la valeur précédente et celle de l'art antérieur, l'aldéhyde remonte et se situe ausi entre la valeur précédente et celle de l'art antérieur.

On peut continuer d'opérer le procédé en reprenant l'introduction de soufre (qu'on avait arrêtée), on constate un retour aux valeurs observées lors de la première introduction du soufre.

La demanderesse a trouvé que le catalyseur à base de métal précieux s'était transformé en un catalyseur à base d'une phase solide de soufre et de métal précieux. La présente invention concerne aussi ce nouveau catalyseur en lui-même. Avantageusement, ce catalyseur est une phase solide de soufre et de palladium déposé sur un support pouvant être du charbon, de la silice, du carbure de silicium ou du carbure de bore.

De préférence ce catalyseur est du $Pd_4S$ déposé sur du charbon.

La présente invention concerne aussi un procédé de deshalogénation d'acides carboxyliques alphahalogénés ou de leurs esters par l'hydrogène, caractérisé en ce que ladite deshalogénation a lieu en présence d'un catalyseur comprenant une phase solide de soufre et d'un métal précieux du groupe VIII.

On peut aussi dans ce procédé utilisant cette phase solide, continuer d'ajouter du soufre avantageusement dans le courant d'acides à deshalogéner. Le soufre se retrouve en sortie dans les acides deshalogénés sous forme de produits éliminables par distillation. Les conditions opératoires sont les mêmes que celles décrites précédemment. Ce procédé utilisant le catalyseur qui est une phase solide soufre métal précieux en conjonction ou non avec une addition de soufre, peut aussi être utilisé pour purifier des acides $R_1CHXCOOH$ et en particulier de l'acide monochloracétique.

## EXEMPLE 1

On charge une colonne A en verre, double enveloppe de 26 mm de diamètre intérieur, avec 300 $cm^3$ d'un charbon extrudé de diamètre 2 mm et longueur 4 mm ayant une surface spécifique > 700 $m^2/g$ et contenant 0,8 % de palladium déposé à la surface (> 100 $m^2/g$ de palladium).

Cette colonne est ensuite alimentée à co-courant par une solution d'acide (en % poids) : acide monochloracétique environ 80 %, acide dichloracétique environ 4 %, acide acétique environ 16 %, et un flux d'hydrogène de 4 normaux litres heure.

La colonne est portée en température, 125°C, et après une certaine période de mise en régime, on procèdera aux variations d'alimentations et on observera les résultats tels que décrits dans le tableau en annexe.

La colonne est alimentée avec un liquide contenant 30 ppm de soufre de la 187° heure à la 355° heure.

Les résultats sont reportés dans le tableau I.

- Les heures de marche désignent les heures cumulées depuis le temps 0.
- VSL désigne la vitesse spaciale liquide.

On a effectué un dosage de soufre dans l'AMCA à la sortie de la colonne à la 350° heure, on a trouvé 29 ppm.

L'analyse par diffraction X de grains de catalyseur prélevés après la 350° heure montre la présence de $Pd_4S$ et l'absence de Pd.

TABLEAU I

| Heures de marche | V.S.L. Kg/h $m^3$ | ADCA sortie % | $CH_3CHO$ formé mg/Kg | Rétrogradation |
|---|---|---|---|---|
| 165 | 245 | 0,12 | 1185 | 3 |
| 187 | 235 | 0,13 | 307 | 1,77 |
| 211 | 234 | 0,22 | 238 | 1,73 |
| 284 | 244 | 0,3 | 242 | 1,87 |
| 355 | 244 | 0,33 | 232 | 1,84 |
| 379 | 243 | 0,25 | 416 | 1,90 |
| 413 | 251 | 0,08 | 656 | 2,30 |
| 442 | 244 | 0,06 | 776 | 2,42 |
| 468 | 244 | 0,08 | 786 | 2,30 |
| 540 | 249 | < 0,03 | 982 | 2,14 |
| ARRET COLONNE | | | | |

**EXEMPLE 2**

On opère comme dans l'exemple 1 avec une colonne B identique à A, mais en utilisant du $S_2Cl_2$ (30 ppm en S) entre la 353° heure et la 616° heure. Les résultats sont reportés dans le tableau II.

## TABLEAU II

| Heures de marche | V.S.L. Kg/h $m^3$ | ADCA sortie % | $CH_3CHO$ formé mg/Kg | Rétrogradation |
|---|---|---|---|---|
| 165 | 247 | 0,21 | 1513 | 3,05 |
| 189 | 241 | 0,16 | 1371 | 3,09 |
| 252 | 243 | 0,12 | 1366 | 3,37 |
| | | 0,16 | 1243 | 3,28 |
| 350 | 249 | 0,07 | 1321 | 2,61 |
| 353 | | | | |
| 374 | 242 | 0,15 | 323 | 1,5 |
| 422 | 234 | 0,20 | 274 | 1,48 |
| 518 | 233 | 0,20 | 295 | 1,40 |
| 568 | 210 | 0,13 | 287 | 1,44 |
| 616 | 215 | 0,17 | 256 | 1,40 |
| 663 | 246 | 0,14 | 690 | 1,91 |
| 758 | 237 | 0,11 | 938 | 2,28 |

Des grains de catalyseur sont prélevés après la 400° heure et, comme dans l'exemple 1, on n'y trouve que du $Pd_4S$.

## EXEMPLE 3

La colonne A est exploitée comme dans l'exemple 1 avec le catalyseur qui a déjà fonctionné dans l'exemple 1 mais entre la 685° et 827° heure, on ajoute 36 ppm de sulfure de carbone à l'AMCA impur. Les résultats sont reportés sur le tableau III.

TABLEAU III

| Heures de marche | V.S.L. Kg/h m³ | ADCA sortie % | $CH_3CHO$ formé mg/Kg | Rétrogradation |
|---|---|---|---|---|
| 590 | 247 | < 0,03 | 951 | 2,25 |
| 685 | 234 | < 0,03 | 1174 | 2,47 |
| 709 | 250 | 0,05 | 307 | 1,56 |
| 755 | 253 | 0,08 | 276 | 1,55 |
| 803 | 236 | 0,06 | 289 | 1,63 |
| 827 | 245 | 0,08 | 247 | 1,63 |

Le soufre dosé en sortie, pendant l'injection de $CS_2$, est d'environ 27 mg/Kg.

## Revendications

1. Procédé de deshalogénation d'acides carboxyliques alphahalogénés ou de leurs esters par l'hydrogène en présence d'un catalyseur à base d'un métal précieux du groupe VIII, caractérisé en ce qu'on effectue ladite deshalogénation en présence de soufre ou de composés du soufre.

2. Procédé selon la revendication 1, caractérisé en ce que les acides carboxyliques alphahalogénés sont de formule (I) :

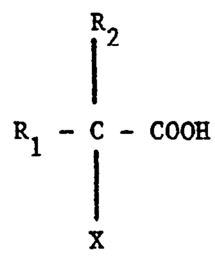

dans laquelle X est le chlore ou le brome, $R_1$ et $R_2$ sont identiques ou différents et représentent X, H, un radical alkyle linéaire ou ramifié ayant de 1 à 12 carbones ou un radical cycloalkyle ayant de 3 à 12 carbones.

3. Procédé selon la revendication 1 ou 2, caractérisé en ce que le métal précieux est du palladium déposé sur du charbon en quantité comprise entre 0,3 à 1 % en poids du catalyseur.

4. Procédé selon l'une des revendications 1 à 3, caractérisé en ce que le composé de soufre est du $S_2Cl_2$ ou du $CS_2$.

5. Procédé selon l'une des revendications 1 à 4, caractérisé en ce que la quantité de soufre ou de composés de soufre est comprise entre 0,1 et 200 mg/Kg de l'acide ou du mélange contenant les acides à hydrogéner, la quantité étant exprimée en soufre.

6. Procédé de purification d'acide monochloracétique contenant de l'acide dichloracétique et éventuellement de l'acide acétique, caractérisé en ce qu'on le traite selon le procédé des revendications 1 à 5.

7. Catalyseur comprenant une phase solide de soufre et d'un métal précieux du groupe VIII.

8. Catalyseur selon la revendication 7, caractérisé en ce qu'il comprend une phase solide de palladium $Pd_4S$ déposée sur du charbon.

9. Procédé de deshalogénation d'acides carboxyliques alphahalogénés ou de leurs esters par hydrogène, caractérisé en ce que ladite deshalogénation a lieu en présence d'un catalyseur selon la revendication 7 ou 8.

10. Procédé selon la revendication 9, caractérisé en ce qu'on effectue la deshalogénation en plus en présence de soufre ou de composés de soufre.

11. Procédé de purification d'acide monochloracétique contenant de l'acide dichloracétique et éventuellement de l'acide acétique, caractérisé en ce qu'on le traite selon le procédé des revendications 9 ou 10.

## Patentansprüche

1. Verfahren zur Dehalogenierung alpha-halogenierter Carboxylsäuren oder deren Ester mittels Wasserstoff in Gegenwart eines Katalysators auf Basis eines Edelmetalls der Gruppe VIII, dadurch gekennzeichnet, daß man die genannte Dehalogenierung in Gegenwart von Schwefel oder Schwefelverbindungen durchführt.

2. Verfahren nach Anspruch 1, dadurch gekennzeichnet, daß die alpha-halogenierten Carboxylsäuren der Formel (I)

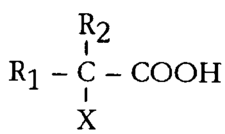

entsprechen, in der X Chlor oder Brom ist, $R_1$ und $R_2$, identisch oder verschieden, X, H, einen linearen oder verzweigten $C_1$- bis $C_{12}$-Alkylrest oder einen $C_3$- bis $C_{12}$-Cycloalkylrest darstellen.

3. Verfahren nach Anspruch 1 oder 2, dadurch gekennzeichnet, daß das Edelmetall Palladium ist, das in einer Menge zwischen 0,3 und 1 Masse-%, bezogen auf den Katalysator, auf Kohle aufgebracht ist.

4. Verfahren nach einem der Ansprüche 1 bis 3, dadurch gekennzeichnet, daß die Schwefelverbindung $S_2Cl_2$ oder $CS_2$ ist.

5. Verfahren nach einem der Ansprüche 1 bis 4, dadurch gekennzeichnet, daß die Menge an Schwefel oder Schwefelverbindungen, berechnet als Schwefel, zwischen 0,1 und 200 mg pro kg Säure oder des Gemisches der zu hydrogenierenden Säuren beträgt.

6. Verfahren zur Reinigung Monochloressigsäure, die Dichloressigsäure und gegebenenfalls Essigsäure enthält, dadurch gekennzeichnet, daß man nach einem der Ansprüche 1 bis 5 verfährt.

7. Katalysator, enthaltend eine feste Phase aus Schwefel und ein Edelmetall der Gruppe VIII.

8. Katalysator nach Anspruch 7, dadurch gekennzeichnet, daß er eine feste Phase von $Pd_4S$ auf Kohle enthält.

9. Verfahren zur Dehalogenierung alpha-halogenierter Carboxylsäuren oder deren Ester mittels Wasserstoff, dadurch gekennzeichnet, daß die genannte Dehalogenierung in Gegenwart eines Katalysators nach dem Anspruch 7 oder 8 erfolgt.

10. Verfahren nach Anspruch 9, dadurch gekennzeichnet, daß man die Dehalogenierung zusätzlich in Gegenwart von Schwefel oder einer Schwefelverbindung durchführt.

11. Verfahren zur Reinigung von Monochloressigsäure, die Dichloressigsäure und gegebenenfalls Essigsäure enthält, dadurch gekennzeichnet, daß man sie nach Anspruch 9 oder 10 behandelt.

## Claims

1. Process for the dehalogenation of alpha-halogenated carboxylic acids or their esters using hydrogen in the presence of a catalyst based on a precious metal of group VIII, characterized in that the said dehalogenation is carried out in the presence of sulphur or of sulphur compounds.

2. Process according to Claim 1, characterized in that the alpha-halogenated carboxylic acids are of formula (I):

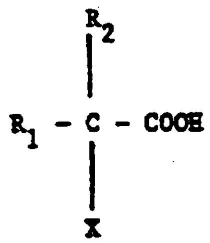

in which X is chlorine or bromine, and $R_1$ and $R_2$ are identical or different and represent X, H, a linear or branched alkyl radical containing from 1 to 12 carbons or a cycloalkyl radical containing from 3 to 12 carbons.

3. Process according to Claim 1 or 2, characterized in that the precious metal is palladium which is deposited on carbon in an amount between 0.3 and 1% by weight of the catalyst.

4. Process according to one of Claims 1 to 3, characterized in that the sulphur compound is $S_2Cl_2$ or $CS_2$.

5. Process according to one of Claims 1 to 4, characterized in that the amount of sulphur or of sulphur compounds is between 0.1 and 200 mg/kg of the acid or of the mixture containing the acids to be hydrogenated, the amount being expressed as sulphur.

6. Process for the purification of monochloroacetic acid containing dichloroacetic acid and possibly acetic acid, characterized in that it is treated according to the process of Claims 1 to 5.

7. Catalyst comprising a solid sulphur phase and a precious metal of group VIII.

8. Catalyst according to Claim 7, characterized in that it comprises a solid palladium phase $Pd_4S$ deposited on carbon.

9. Process for the dehalogenation of alpha-halogenated carboxylic acids or their esters using hydrogen, characterized in that the said dehalogenation takes place in the presence of a catalyst according to Claim 7 or 8.

10. Process according to Claim 9, characterized in that the dehalogenation is also carried out in the presence of sulphur or of sulphur compounds.

11. Process for the purification of monochloroacetic acid containing dichloroacetic acid and possibly acetic acid, characterized in that it is treated according to the process of Claim 9 or 10.